# EUROPEAN PATENT APPLICATION

(11) **EP 4 014 748 A1**
(43) Date of publication of application: **22.06.2022**
(21) Application number: 20215900.0
(22) Date of filing: 21.12.2020
(51) Int. Cl.: A23K 10/16, A23K 10/30, A23K 20/189, A23K 50/75, C12N 1/14, C12P 19/14

(54) **SECRETOMES OF ASPERGILLUS STRAINS FOR THE DEGRADATION OF SOYBEAN MEAL**

(71) Applicant: Adisseo France S.A.S., 92160 Antony (FR); Institut national de recherche pour l'agriculture, l'alimentation et l'environnement, 75007 Paris (FR); Université d'Aix Marseille, 13007 Marseille (FR)
(72) Inventor: BERRIN, Jean-Guy, 13360 Roquevaire (FR); NAVARRO, David, 13009 Marseille (FR); GRANDMONTAGNE, Delphine, 13006 Marseille (FR); NEUGNOT, Virginie, 31100 Toulouse (FR)
(74) Representative: Germain Maureau

(57) **Abstract**

The present invention relates to the use of a secretome of an *Aspergillus* strain, said strain belonging to the speciesA. *terreus* or *A. japonicus,* or of a composition comprising such a secretome, to improve degradation and/or solubilization of soybean meal, and/or to improve digestibility of soybean meal by an individual.

## Description

### FIELD OF THE INVENTION

The present invention relates to the use of a secretome of an *Aspergillus* strain, said strain belonging to the species *A*. *terreus* or *A. japonicus,* or of a composition comprising such a secretome, to improve degradation and/or solubilization of soybean meal, and/or to improve digestibility of soybean meal by an individual.

### BACKGROUND OF THE INVENTION

Since the middle of the twentieth century, the human population has tripled reaching seven billions today and probably nine billions by 2050, thus leading to increasing challenges in human nutrition (food), as well as animal nutrition (feed). A promising approach is to use by-products from agriculture such as rapeseed, sunflower and soybean meals, since they represent a rich source of proteins.
In particular, soybean meal is the most important protein source used to feed farm animals, particularly in the poultry and swine industries. It can also be used for soy flour and other products for human consumption. Soybean meals are mostly composed of polysaccharides and proteins embedded in a plant cell wall matrix ; cell wall carbohydrates are composed of free sugars (such as sucrose, stachyose and raffinose), as well as cellulose and pectins. However, soybean meal is not well digested, in particular by poultry, since their digestive tracts do not have the endogenous enzymes required to degrade the polysaccharides contained in soybean meal. This can lead to digestive disorders, gas accumulation, diarrhea and lack of nutrient, thus decreasing the yield of meat production.
It has been previously shown that the fermentation of soybean meal by some strains (such as *Aspergillus oryzae* strains) can improve its nutritional quality (Hong et al (2004), J Med Food 7(4) : 430-435 ; Feng et al (2007), Animal Feed Science and Technology 134 : 235-242). Moreover, exogenous enzymes (e.g. cellulases, phytases, xylanases, pectinases, proteases) have been used to supplement endogenous enzymes secreted by the digestive tract of the animal. The use of such enzymes allows an increase of the production of meat per animal, therefore decreasing the time and cost of production, especially in poultry production system. For instance, WO95/28850 discloses a method for improving the solubility of vegetable proteins (such as soybean meal), using a combination of several enzymes (i.e. phytases, proteolytic enzymes and lipolytic or glycosidase enzymes).
Microorganisms secrete into the extracellular space a set of factors, in particular proteins (including enzymes), which are collectively referred to as the secretome of said microorganism. The use of microbial secretomes may present some advantages over the use of individual enzymes, due to the variety of the enzymes they comprise. However, the various enzymes contained in secretomes may exhibit synergistic effects, but potentially also antagonist effects. Therefore, identifying particular secretomes which have particular properties is a complex task. The secretome of fungi is known to comprise a rich panel of enzymes, which may be able to degrade complex biomass. The composition of the secretome and its capacity to degrade various biomasses, depend on the fungal species. For instance, the capacity of filamentous fungi to degrade lignocellulosic biomass has been studied in a range of basidiomycetes and ascomycetes (Lesage-Meessen et al (2002), Appl Biochem Bioetchnol 102-103: 141-153 ; Couturier et al (2012), BMC Genomics 13 : 57 ; Berrin et al (2012), Appl Environ Microbiol 78 : 6483-6490). Moreover, fungal secretomes have allowed the development of enzymatic cocktails in industry in the animal feed sector. The Rovabio^{®} is an enzymatic cocktail produced from the ascomycete fungus *Talaromyces versatilis.* Although this cocktail is efficient to enhance wheat broiler feed digestibility, soybean meal-containing diets are more challenging and there is a need to develop enzymatic cocktails with improved ability to degrade and/or solubilize soybean meal, and/or to improve digestibility of soybean meal by an individual.

It has now been discovered, completely unexpectedly, that a secretome of an *Aspergillus terreus* or *an Aspergillus japonicus* strain can achieve these aims. Moreover, these secretomes can advantageously be used in combination with the Rovabio^{®} enzymatic cocktail.

### DESCRIPTION OF THE INVENTION

Therefore, the present invention relates to the use of :
- a secretome of an *Aspergillus* strain, said strain belonging to the species *A*. *terreus* or *A. japonicus,* or
- a composition comprising such a secretome,
to improve degradation and/or solubilization of soybean meal, and/or to improve digestibility of soybean meal by an individual.

In the context of the present invention:
- Soybean meal is the by-product (which appears as a solid residue) of the extraction of soybean oil. Soybean oil and soybean meal can be obtained by several methods. The most common process to extract soybean oil consists in extracting oil from soybean flakes by solvent. A second method consists in a mechanical extraction by a screw press (expeller). A third method combines extruding and expelling of soybean flakes, and uses solvent for oil extraction ;
- The term "individual" refers to a human or an animal;
- The "secretome" of an organism represents the set of proteins (including enzymes) secreted by said organism into the extracellular space (in particular, into the culture medium) ;
- The "composition" can in particular be a food or feed composition. It can be used as a supplement or additive.
- "Improving degradation of soybean meal" means increasing the degradation of one or several of the components of soybean meal into smaller parts. For instance, it includes the degradation/modification of proteins of soybean meal into smaller proteins, peptides or amino acids, and/or the degradation/modification of carbohydrates of soybean meal into smaller sugars. Degradation of soybean meal can for instance be measured as described in the examples ;
- "Improving solubilization of soybean meal" means that soybean meal is made more soluble in aqueous solution. This can for instance be measured based on the residual dry matter, as described in the examples ;
- "Improving digestibility of soybean meal by an individual" means that soybean meal is rendered more digestible by said individual, as can for instance be assessed by measurement of apparent metabolizable energy (AME) and ileal amino acids in animal fed with soybean meal-based diet.

Preferably, the present invention relates to the use of a secretome or composition as described above, wherein the *Aspergillus* strain is selected in the group consisting of A. *terreus* CIRM BRFM 111 (deposited under the Budapest treaty on June 25, 2020 at Collection Nationale de Cultures de Micro-organismes (CNCM), 25 rue du Docteur Roux, Paris, France, as CNCM 1-5526) and *A. japonicus* CIRM BRFM 405 (deposited under the Budapest treaty on June 6, 2012 at CNCM, as CNCM 1-4639).

Preferably, the present invention relates to the use of a secretome or composition as described above, wherein the secretome of the *Aspergillus* strain is obtainable by culturing said *Aspergillus* strain in presence of a substrate comprising pectin, more preferably at least 5, 6, 7, 8, 9, 10, 11, 12, 13, 14, 15, 16, 17, 18, 19, 20 % of pectin. Examples of such substrates include sugar beet, soybean, rapeseed and citrus, as well as parts or extracts thereof (such as sugarbeet pulp, soybean meal, rapeseed meal or citrus pectin). More preferably, the substrate is sugar beet pulp and/or soybean meal, even more preferably sugar beet pulp. Sugarbeet pulp is the fibrous, energy rich by-product resulting from the water extraction of sugar contained in the root of the sugarbeet (*Beta vulgaris* L.). The substrate may be used between 5 and 30 g of dry matter per liter of culture medium (e.g. 5, 10, 15, 20, 25 or 30 g/L). The substrate may for instance be in the form of particles, which can be of various sizes, in particular between 30 µm and 3 mm. For instance, the substrate may be milled, grinded or micronized.

Apart from the substrate, other components of the culture medium of the *Aspergillus* strain are usual and known to the skilled person. For instance, these components may include a carbon source, a nitrogen source, salts, oligo-elements, vitamins and yeast extract.

In order to obtain the secretome as described above, the *Aspergillus* strains can be cultured at various ranges of temperature (e.g. between 25 and 33°C, such as 25, 26, 27, 28, 29, 30, 31, 32 or 33°C) and pH (e.g. between 3 and 7, such as a pH of 3, 4, 5, 6 or 7) during various times (e.g. between 3 and 10 days, such as during 3, 4, 5, 6, 7, 8, 9 or 10 days). The *Aspergillus* strains can for instance be cultured for about 7 days at a temperature of about 30°C and a pH of about 5. Moreover, the *Aspergillus* strains can be cultured in various culture formats (e.g. in flasks, in Erlenmeyer or in bioreactors, using various conditions of agitation and aeration), such as those described in the examples. Secretomes can be obtained after culturing the *Aspergillus* strains, by merely separating cells from the culture supernatant, the latter containing secreted proteins of the secretome. This supernatant can be used directly, or cell debris may be removed, e.g. by filtration. The supernatant may also be concentrated, e.g. by diafiltration/dialysis. Proteins of the secretome may also be retrieved by precipitation in ammonium sulfate.

Preferably, the present invention relates to the use of a secretome or composition as described above, wherein the composition further comprises a secretome of a *Talaromyces versatilis* strain. For instance, said *Talaromyces versatilis* strain may be selected in the group consisting of the strains IMI 134755, IMI 134756 and IMI 378536. Methods for producing secretomes of *T. versatilis* strains are known to the skilled person ; as examples, we can cite the methods described in EP0976838. For instance, Rovabio^{®} Excel and Rovabio^{®} Advance contain secretomes of *Talaromyces versatilis* strains.

Preferably, the present invention relates to the use of a secretome or composition as described above, wherein the composition further comprises at least a protease, at least a phytase and/or at least a carbohydrate-active enzyme.

Preferably, the present invention relates to the use of a secretome or composition as described above, wherein the individual is an animal, more preferably a monogastric animal, even more preferably an animal selected in the group consisting of swine and poultry.

The present invention also relates to a secretome of an *Aspergillus* strain selected in the group consisisting of *A. terreus* CIRM BRFM 111 and *A. japonicus* CIRM BRFM 405, said secretome being obtainable by culturing said strain in presence of a substrate comprising pectin, more preferably at least 5, 6, 7, 8, 9, 10, 11, 12, 13, 14, 15, 16, 17, 18, 19, 20 % of pectin. Examples of such substrates include sugar beet, soybean, rapeseed and citrus, as well as parts or extracts thereof (such as sugarbeet pulp, soybean meal, rapeseed meal or citrus pectin). More preferably, the substrate is sugar beet pulp and/or soybean meal, even more preferably sugar beet pulp. The substrate may for instance be in the form of particles, which can be of various sizes, in particular between 30 µm and 3 mm. For instance, the substrate may be milled, grinded or micronized.

The present invention also relates to a composition comprising :
- a secretome of an *Aspergillus* strain belonging to the species *A. terreus* or *A. japonicus* (preferably selected in the group consisting of *A. terreus* CIRM BRFM 111 and *A. japonicus* CIRM BRFM 405), said secretome being preferably obtainable by culturing said strain in presence of a substrate comprising pectin, preferably at least 5, 6, 7, 8, 9, 10, 11, 12, 13, 14, 15, 16, 17, 18, 19, 20 % of pectin, more preferably sugar beet pulp or soybean meal, even more preferably sugar beet pulp, and
- a secretome of a *Talaromyces versatilis* strain (for instance, said strain may be selected in the group consisting of the strains IMI 134755, IMI 134756 and IMI 378536).

Preferably, the composition as described above further comprises at least a protease, at least a phytase and/or at least a carbohydrate-active enzyme.

Preferably, the composition as described above further comprises soybean meal.

### BRIEF DESCRIPTION OF THE DRAWINGS

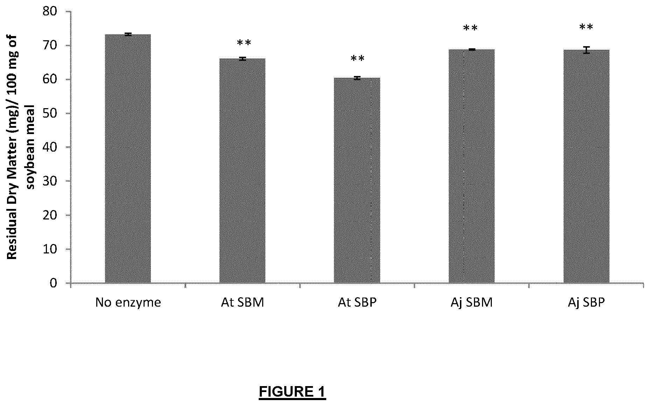

Figure 1: Ability of each secretome to hydrolyze soybean meal. For each condition, 0.1 mg of enzyme of each secretome was added. At: *Aspergillus terreus,* Aj: *Aspergillus japonicus,* SBP: Sugar Beet Pulp, SBM: SoyBean Meal. Significance of the results between "No enzyme" and secretome addition was assessed using t-test (n=3) with ** p value < 0.01.

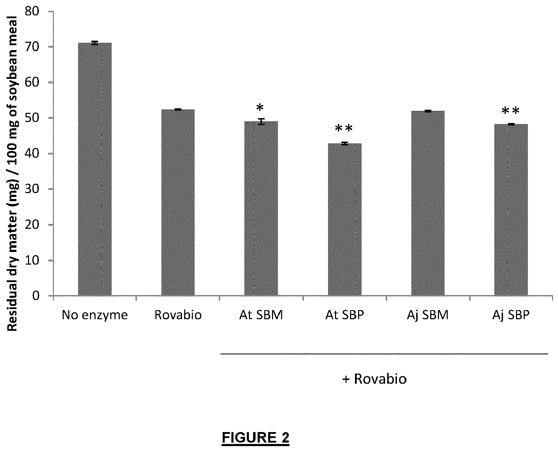

Figure 2: Ability of each secretome to supplement the Rovabio^{®} enzymatic cocktail. Rovabio^{®} (0.9 mg of enzyme) was supplemented with 0.4 mg of enzyme from the produced secretomes. At: *Aspergillus terreus,* Aj: *Aspergillus japonicus,* SBP : Sugar Beet Pulp, SBM: SoyBean Meal. Significance of the results between Rovabio^{®} condition and Rovabio^{®} supplementation with secretome was assessed using t-test (n=3) with p value: *, p value < 0.05; ** p value < 0.01.

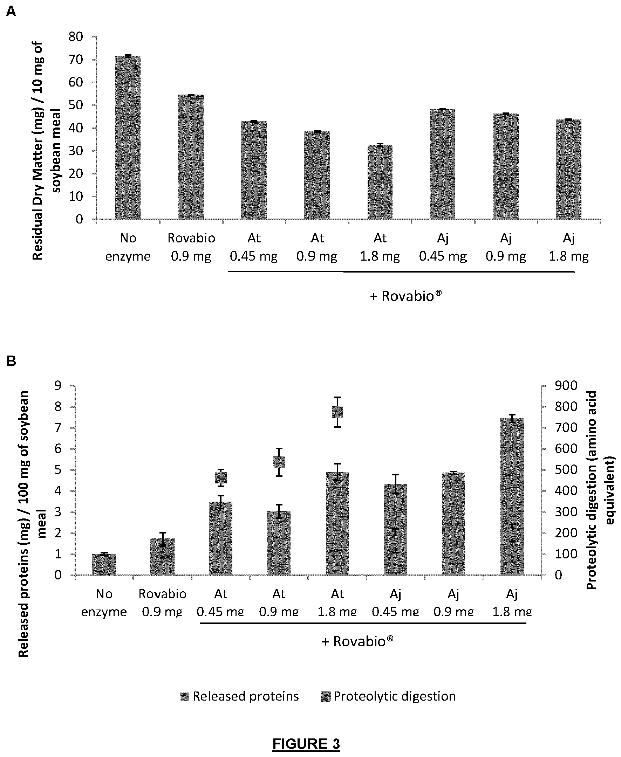

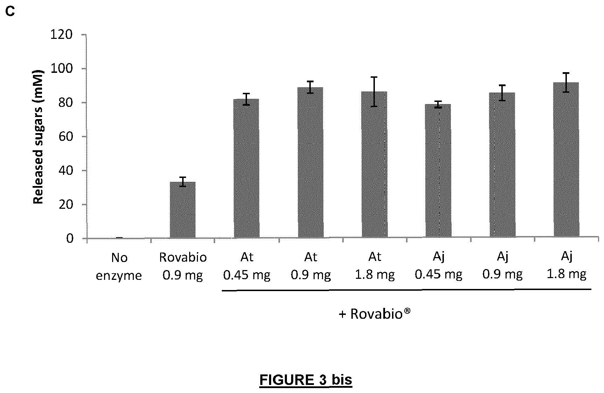

Figure 3: Effect of the *Aspergilli* secretomes on the protein and sugar release from soybean meal. A. Residual dry matter after supplementation of Rovabio^{®} with different amounts of enzymes for *Aspergillus terreus* (At) and *Aspergillus japonicus* (Aj) secretomes. B. Total amount of released proteins (histograms) and proteolytic digestion (small squares) after supplementation of Rovabio^{®} with different amounts of enzymes for *Aspergillus terreus* (At) and *Aspergillus japonicus* (Aj) secretomes. C. Total amount of released sugars after supplementation of Rovabio^{®} with different amounts of enzymes from *A. terreus* (At) and *A. japonicus* (Aj) secretomes.

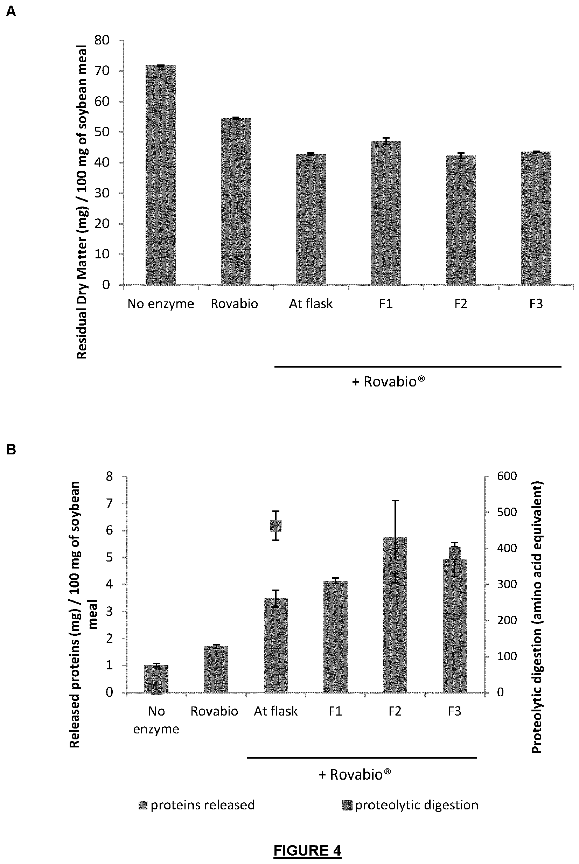

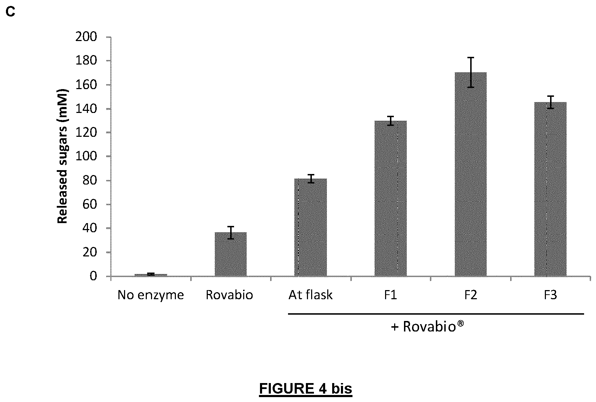

Figure 4: Effect of the *Aspergilli* secretomes produced in bioreactor on the protein and sugar release from soybean meal. A. Residual dry matter after soybean meal hydrolysis. F1, F2 and F3 are secretomes of *Aspergillus terreus* produced in bioreactors. B. Total amount of released proteins (histograms) and proteolytic digestion (small squares) after supplementation of Rovabio^{®} with *A. terreus* secretomes produced in flask. C. Soluble sugars released after soybean meal hydrolysis by the Rovabio^{®} supplemented with the *A. terreus* secretomes produced in bioreactor. Error bars represent standard deviation between triplicates.

### EXAMPLES

The present invention is illustrated non-exhaustively by the following examples. These examples are intended for the purpose of illustration only and are not intended to limit the scope of the present invention.

### Example 1 : Secretome production from Aspergillus strains

The fungal strains used were *Aspergillus japonicus* BRFM 405 (deposited under the Budapest treaty as CNCM I-4639) and *Aspergillus terreus* BRFM 111 (deposited under the Budapest treaty as CNCM I-5526), both of the phyllum Ascomycota and of the family Trichocomaceae. These strains were from the CIRM-CF (*Centre International de Ressources Microbiennes-Champignons Filamenteux,* INRAE, Marseille, France, https://www6.inrae.fr/cirm eng/Filamentous-Fungi) collection, and were authenticated using molecular markers.

Secretome from each *Aspergillus* strain was prepared following previous observations (Lesage-Meessen et al (2002), Appl Biochem Bioetchnol 102-103: 141-153). Briefly, *Aspergillus* strains were cultivated on Potato Dextrose Agar (PDA) solid medium, and after 10 days of growth they were used to produce the secretome. The spores of *Aspergillus* were used directly to inoculate the culture media (2x10⁵ spores/mL). Fungal cultures were grown in 500 mL baffled flasks, in a 100 mL liquid medium containing 15 g/L (based on the dry matter) of autoclaved biomass (micronized sugar beet pulp (SBP), or micronized soybean meal (SBM)) as a carbon source, 2.5 g/L of maltose as a starter, 1.842 g/L of diammonium tartrate as a nitrogen source, 0.5 g/L yeast extract, and salts (KH₂PO₄: 0.2 g/L; CaCl₂: 0.0132 g/L; MgSO₄: 0.5 g/L). Flasks were incubated at 30°C at a pH of about 5, with orbital shaking at 120 rpm. After 7 days of inoculation, the culture broths (secretomes) were harvested and pooled (total volume 300 mL per conditions). The supernatant was filtered on a 10 kDa pore sized membrane (vivaspin polyethersulfone, Sartorius, Göttingen, Germany), and concentrated in a sodium acetate buffer (pH 5, 50 mM) to a final volume of 10 mL. They were stored at -20°C until use.

### Example 2 : The secretomes of the Aspergillus strains are able to solubilize soybean meal

To assess the efficiency of each secretome on the solubilization of soybean meal, the residual dry matters obtained after incubating soybean meal with the different secretomes, were measured. Briefly, 150 mg of micronized soybean meal were hydrolyzed with 0.1 mg of enzyme obtained from the various secretomes, diluted in 1 mL sodium acetate buffer (pH 4; 100 mM). The samples were incubated 24 h at 37°C, with an orbital shaking at 850 rpm. At the end of the reaction, 1 mL of KOH 0.34% (w/v) was added and samples were shaken on digital tube revolver at 20 rpm (ThermoFisher scientific, Waltham, United States) during 20 minutes. Samples were centrifuged at 4°C, 10,000 rpm during 15 minutes. The supernatant was collected for subsequent use. The pellets were washed three times with water, and dried at 100°C to measure the dry matter.

As shown in Figure 1, the secretomes of the *Aspergillus terreus* and the *Aspergillus japonicus* strains, whether grown on SBM or SBP, were able to significantly improve solubilization of soybean meal.

### Example 3 : The secretomes of the Aspergillus strains in combination with the Rovabio^{®} cocktail, improve the solubilization of soybean meal

The various secretomes (0.4 mg of proteins, as quantified using the Bradford assay) were then used in combination with the Rovabio^{®} Advance enzymatic cocktail (0.9 mg of proteins, as quantified using the Bradford assay) (hereafter, "Rovabio") and the effect on the solubilization of soybean meal was assessed, as described above, in comparison with the effect of Rovabio^{®} alone.

As shown in Figure 2, the various secretomes in combination with Rovabio^{®} significantly improved the solubilization of soybean meal, compared to Rovabio^{®} alone. The combination of Rovabio^{®} with the secretome of *A. terreus* grown on SBP was particularly efficient (decrease of 10% of the residual dry matter, compared to the use of Rovabio^{®} alone).

### Example 4 : Effect of the secretomes of the Aspergillus strains at different amounts, in combination with Rovabio^{®}, on the solubilization of soybean meal, and on the protein and sugar release from soybean meal

Increasing amounts (0.45, 0.9 or 1.8 mg of proteins, as quantified using the Bradford assay) of each secretome of *A. terreus* and *A. japonicus* grown on SBP were supplemented to Rovabio^{®} (0.9 mg of proteins, as quantified using the Bradford assay), and incubated with soybean meal, as described previously. Supernatants were collected as described in Example 2, and used to perform various tests. The release of soluble reducing sugars was quantified using the dinitrosalicylic acid (DNS) assay (Reference 128848, Sigma-Aldrich), proteins release was quantified using the Bradford assay (Reference 5000006, Bio-Rad), and proteolytic digestion was evaluated with the TNBS (2,4,6-Trinitrobenzenesulfonic acid) assay (Reference P2297, Sigma-Aldrich). The significance of the results between Rovabio^{®} condition and Rovabio^{®} supplementation with secretome was assessed using t-test (n=3).

With the increasing addition of enzymes from each secretome, the residual dry matter decreased compare to Rovabio^{®} alone (Figure 3A). The boosting effect was more striking with the secretome of *A. terreus* compared to the one of *A. japonicus* with respectively 32% and 43% of residual dry matter after hydrolysis.

Moreover, the supplementation of Rovabio^{®} with the different secretomes of *Aspergilli* increased the release of proteins. The TNBS assay revealed that the *A. terreus* secretome cleaved the released proteins into smaller peptides compared to the *A*. *japonicus* secretome (Figure 3B).

The supplementation of Rovabio^{®} with the different secretomes of *Aspergilli* also increased the release of soluble sugars (Figure 3C). However, the DNS method evaluates the reducing ends but does not give any information about the size of the released sugars. The increase in released sugars suggests that the number of cleavages in soybean meal polysaccharides is higher in supplementation conditions but does not give any information on the nature of the sugars released.

In order to evaluate the monomeric sugar composition of polysaccharides, pellets were hydrolyzed with sulfuric acid (76%, 30 min, 30°C). Another sulfuric acid hydrolysis was performed (1 M, 100°C, 2 h). Neutral sugars were derivatized into alditol acetate and analyzed on gas phase chromatography (Englyst and Cummings (1988), J Assoc Off Anal Chem 71 : 808-814). Acidic sugars were evaluated with the Skalar system, using the colorimetric MHDP method (Blumenkrantz and Asboe-Hansen (1973), Analytical Biochemistry 54 : 484-489).

Table 1 shows compositional analysis of the sugar monomers (rhamnose, fucose, arabinose, xylose, mannose, galactose, glucose and uronic acids) present in the residual fraction of the soybean meal after enzymatic hydrolysis by Rovabio^{®} supplemented or not by the secretomes of *A. japonicus* or *A. terreus.* The most striking effect was observed with the supplementation of the secretome of *A. terreus* that decreased the amount of fucose, arabinose, xylose, galactose, glucose and uronic acids compared to Rovabio^{®} alone.

**Table 1. Compositional analysis of the sugar content of the residual dry matter after hydrolysis. Soybean meal was hydrolyzed with Rovabio^{®}, supplemented or not with Aspergillus terreus (At) secretome grown on SBP (0.45 mg of enzyme), or Aspergillus japonicus (Aj) grown on SBP (0.45 mg of enzyme). The sugar composition of the residual dry matter was analyzed. Rha: rhamnose, Fuc: fucose, Ara: arabinose, Xyl: Xylose, Man: mannose, Gal: galactose, Glc: glucose, UA: uronic acids. Significance of the results between Rovabio^{®} condition and Rovabio^{®} supplementation with secretome was assessed using t-test (n=3) with p value: *, p value < 0.05; **, p value < 0.01.**

| | **Massic %** | | | |
|---|---|---|---|---|
| | No Enzyme | Rovabio^{®} | Rovabio^{®} + Aj | Rovabio^{®} + At |
| **Rha** | 0.388 ± 0.048 | 0.315 ± 0.007 | 0.375 ± 0.026 | 0.315 ± 0.071 |
| **Fuc** | 0.444 ± 0.097 | 0.450 ± 0.028 | 0.414 ± 0.360 | 0.298 ± 0.061* |
| **Ara** | 3.659 ± 0.153 | 1.644 ± 0.199 | 1.030 ± 0.065 | 0.882 ± 0.106** |
| **Xyl** | 2.073 ± 0.104 | 1.909 ± 0.070 | 2.220 ± 0.218 | 1.158 ± 0.269* |
| **Man** | 1.461 ± 0.040 | 1.136 ± 0.102 | 0.915 ± 0.122 | 0.853 ± 0.165 |
| **Gal** | 5.736 ± 0.228 | 1.742 ± 0.082 | 1.231 ± 0.104** | 0.794 ± 0.078** |
| **Glc** | 8.532 ± 0.336 | 6.531 ± 0.841 | 5.288 ± 0.249 | 3.468 ± 0.544** |
| **UA** | 5.258 ± 0.437 | 5.687 ± 0.206 | 7.154 ± 0.097 | 4.163 ± 0.334** |

All these results tend to show that the effect on solubilization of soybean meal observed with the various secretomes may result from the degradation of soybean meal (in particular, degradation of the proteins and the carbohydrates contained in soybean meal).

### Example 5 : Efficiency of Aspergillus secretomes produced in bioreactor

To upscale and validate the supplementation effect of the *A. terreus* secretome, a bioreactor production was carried out. Briefly, *Aspergillus terreus* cultures were performed in 2L bioreactors, in a 1.5L liquid medium containing 15 g/L (based on the dry matter) of autoclaved biomass (micronized or 3-mm particles sized sugar beet pulp) as a carbon source, 2.5 g/L of maltose as a starter, 1.842 g/L of diammonium tartrate as a nitrogen source, 0.5 g/L yeast extract, and salts (KH₂PO₄: 0.2 g/L; CaCl₂: 0.0132 g/L; MgSO₄: 0.5 g/L), 0.05 g/L Tween 80 and anti-foam. Micronized sugar beet pulp was obtained by two successive levels of grinding using an impact mill (Netzsch): the first one with a pin disk (5 mm of diameter), 14000 rotations/min; the second one with a blast rotor, 12000 rotations/min. Bioreactors were incubated at 30°C, with 8 L/h O₂ and orbital shaking at 120 rpm (marine propellers). Three different culture conditions in bioreactors (F1, F2 and F3) were tested, with some differences in the inoculation of spores and the size of the sugar beet pulp particles, as described in Table 2.

**Table 2. Presentation of the different tested parameters, for the culture in 2L bioreactors**

| **Bioreactor** | **Inoculum** | **Sugar Beet Particles Size** |
|---|---|---|
| **F1** | 2.10⁸ spores/L | 3 mm |
| **F2** | 2.10⁸ spores/L | Micronized (<0,1 mm) |
| **F3** | 20.10⁸ spores/L | Micronized (<0,1 mm) |

After 7/10 days of inoculation, the culture broths (secretomes) were harvested. The supernatant was filtered on a 10 kDa pore sized membrane (Vivaspin polyethersulfone, Sartorius), and concentrated in a sodium acetate buffer (pH 5, 50 mM) to a final volume of 10 mL. They were stored at -20°C until use.
The secretomes produced were collected and tested for their ability to supplement the Rovabio^{®} cocktail as previously described.

All secretomes of *A. terreus* produced in bioreactor were able to improve soybean meal solubilization to the same extent compared to the Rovabio^{®} supplemented by *A*. *terreus* produced in flask (Figure 4A). Of note, the supplementation of *A. terreus* secretomes produced in bioreactor F3 released more proteins (Figure 4B) than the secretome produced in flask. Contrary to secretomes produced in bioreactor, the secretome produced in flask cleaved proteins in more peptides. Supplementation of Rovabio^{®} with the *A. terreus* secretomes produced in bioreactors released more sugars than the secretome produced in flasks (Figure 4C).

These results show that the effects observed using bioreactors are similar to the effects observed using flasks.

## Claims

1. Use of :
- a secretome of an *Aspergillus* strain, said strain belonging to the species *A*. *terreus* or *A. japonicus,* or
- a composition comprising such a secretome,
to improve degradation and/or solubilization of soybean meal, and/or to improve digestibility of soybean meal by an individual.

2. Use according to claim 1, wherein the *Aspergillus* strain is selected in the group consisting of *A. terreus* CIRM BRFM 111 (deposited under the Budapest treaty as CNCM I-5526) and *A. japonicus* CIRM BRFM 405 (deposited under the Budapest treaty as CNCM I-4639).

3. Use according to claim 1 or 2, wherein the secretome of the *Aspergillus* strain is obtainable by culturing said *Aspergillus* strain in presence of a substrate comprising pectin.

4. Use according to any of the preceding claims, wherein the secretome of the *Aspergillus* strain is obtainable by culturing said *Aspergillus* strain in presence of sugar beet pulp or soybean meal, preferably sugar beet pulp.

5. Use according to any of the preceding claims, wherein the composition further comprises a secretome of a *Talaromyces versatilis* strain.

6. Use according to claim 5, wherein the *Talaromyces versatilis* strain is selected in the group consisting of the strains IMI 134755, IMI 134756 and IMI 378536.

7. Use according to any of the preceding claims, wherein the composition further comprises at least a protease, at least a phytase and/or at least a carbohydrate-active enzyme.

8. Use according to any of the preceding claims, wherein the individual is an animal, preferably a monogastric animal.

9. Use according to any of the preceding claims, wherein the individual is an animal selected in the group consisting of swine and poultry.

10. A secretome of an *Aspergillus* strain selected in the group consisisting of *A*. *terreus* CIRM BRFM 111 and *A. japonicus* CIRM BRFM 405, said secretome being obtainable by culturing said strain in presence of a substrate comprising pectin, preferably sugar beet pulp or soybean meal, more preferably sugar beet pulp.

11. A composition comprising :
- a secretome of an *Aspergillus* strain belonging to the species *A. terreus* or *A. japonicus,* said secretome being preferably obtainable by culturing said strain in presence of a substrate comprising pectin, more preferably sugar beet pulp or soybean meal, even more preferably sugar beet pulp, and
- a secretome of a *Talaromyces versatilis* strain.

12. A composition according to claim 11, wherein the *Aspergillus* strain is selected in the group consisting of *A. terreus* CIRM BRFM 111 and *A. japonicus* CIRM BRFM 405.

13. A composition according to claim 11 or 12, wherein the *Talaromyces versatilis* strain is selected in the group consisting of the strains IMI 134755, IMI 134756 and IMI 378536.

14. A composition according to any of claims 11 to 13, further comprising at least a protease, at least a phytase and/or at least a carbohydrate-active enzyme.
